# EUROPÄISCHE PATENTSCHRIFT

(11) **EP 1 827 384 B1**
(45) Veröffentlichungstag und Bekanntmachung des Hinweises auf die Patenterteilung: **10.04.2013**
(21) Anmeldenummer: 05849696.9
(22) Anmeldetag: 21.12.2005
(51) Int. Cl.: A61K 9/20, A61K 31/404, A61P 9/12, A61K 31/18

(54) **DIREKT VERPRESSTE INDAPAMID-TABLETTEN MIT VERZÖGERTER WIRKSTOFFFREISETZUNG**
DIRECTLY PRESSED INDAPAMIDE TABLETS WITH DELAYED RELEASE OF THE ACTIVE SUBSTANCE
COMPRIMES D'INDAPAMIDE A LIBERATION DIFFEREE DU PRINCIPE ACTIF OBTENUS PAR COMPRESSION DIRECTE

(30) Priorität: 23.12.2004 DE 102004062257
(43) Veröffentlichungstag der Anmeldung: 05.09.2007
(73) Patentinhaber: MERCKLE GMBH, 89079 Ulm (DE)
(72) Erfinder: SWATSCHEK, Dieter, 89143 Blaubeuren (DE); SIEVERT, Frank, 89604 Allmendingen (DE); STREIL, Frank, 89143 Blaubeuren (DE)
(74) Vertreter: Best, Michael
(86) Internationale Anmeldenummer: PCT/EP2005/013796
(87) Internationale Veröffentlichungsnummer: WO 2006/069705

(56) Entgegenhaltungen:
- EP-A- 0 519 820
- EP-A- 1 057 479
- WO-A-2004/002475
- WO-A-2005/074884
- US-B1- 6 653 336
- DAMIEN G ET AL: "GALENIC DEVELOPMENT AND PHARMACOKINETIC PROFILE OF INDAPAMIDE SUSTAINED RELEASE 1.5MG" CLINICAL PHARMACOKINETICS, LEA & FEBIGER, PHILADELPHIA, PA, US, Bd. 37, Nr. SUPPL 1, 1999, Seiten 13-19, XP009004369 in der Anmeldung erwähnt
- GUEZ, D.: "L'indapamide LP 1,5mg: apport d'un diurétique de nouvelle génération pour le traitement de l'hypertension répondant aux recommandations internationales" DISEASE MANAGEMENT AND HEALTH OUTCOMES, Bd. 5, 1999, Seiten 21-30, XP008062197
- SCHIAVI, PIERRE: "Pharmacokinetics of sustained and immediate release formulations of indapamide after single and repeated oral administration in healthy volunteers" FUNDAM. CLIN.PHARMACOL., Bd. 14, 2000, Seiten 139-146, XP008062198
- CUI ET AL: "Development of indapamide sustained-release tablets and study on its release mechanism", ZHONGGUO YIYUAN YAOXUE ZAZHI - CHINESE JOURNAL OF HOSPITALPHARMACY, ZHONGGUO YAO XUEHUI WUHAN FENHUI, WUHAN, CN, vol. 23, no. 7, 1 July 2003 (2003-07-01), pages 397-399, XP009102755, ISSN: 1001-5213
- GOHEL M C ET AL: "A review of co-processed directly compressible excipients", JOURNAL OF PHARMACY AND PHARMACEUTICAL SCIENCES, CANADIAN SOCIETY FOR PHARMACEUTICAL SCIENCES, EDMONTON, CA, vol. 8, no. 1, 1 January 2005 (2005-01-01) , pages 76-93, XP009131575, ISSN: 1482-1826

## Beschreibung

Die Erfindung betrifft pharmazeutische Zusammensetzungen, welche den Wirkstoff Indapamid enthalten und zur Herstellung von Tabletten mit verzögerter Wirkstofffreisetzung durch Direktverpressung geeignet sind, so dass auf eine vorhergehende Granulierung verzichtet werden kann. Ferner betrifft die Erfindung Tabletten mit verzögerter Wirkstofffreisetzung, welche durch Direktverpressung dieser pharmazeutischen Zusammensetzungen erhältlich sind.

Indapamid, 4-Chlor-N-(2-methylindolin-1-yl)-3-sulfamoyl-benzamid, hat die chemische Strukturformel (I) 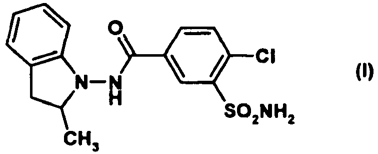 und ist ein Sulfamidderivat mit antihypertensiver Wirkung. Es wird insbesondere zur Behandlung essentieller Hypertonie eingesetzt.

Im Stand der Technik sind Darreichungsformen von Indapamid bekannt, welche den Wirkstoff im wesentlichen ohne zeitliche Verzögerung freisetzen. EP-A 1 057 479 offenbart Arzneimittelformulierungen, die zur Herstellung von direktverpressten Tabletten mit schneller Wirkstofffreisetzung geeignet sein sollen. Neben mehreren weiteren Wirkstoffen wird in einem Beispiel auch Indapamidhemihydrat als Wirkstoff eingesetzt. Gemäß der EP-A 1 057 479 muss in solchen Formulierungen ein Inhibitor vorhanden sein, der eine Wechselwirkung zwischen dem pharmazeutischen Inhaltsstoff und den Hilfsstoffen zur Direktverpressung verhindert. Formulierungen, die zur Herstellung von Tabletten mit verzögerter Wirkstofffreisetzung geeignet sind, sind in der EP-A 1 057 479 nicht offenbart. Eine Verabreichung von Indapamid mit sofortiger Freisetzung hat jedoch Nachteile, da sie bei bestimmten Patienten zu kurzfristig sehr hohen Plasmakonzentrationen führen kann.

Im allgemeinen bewirken nichtretardierte perorale oder parenterale Darreichungsformen nach einmaliger Verabreichung den schnellen Aufbau von Plasmaspiegeln. Nach beendeter Resorption kommt es jedoch je nach Größe der Eliminationskonstante zu einem mehr oder weniger schnellen Abfall der Plasmakonzentration des Wirkstoffs. Dies führt bei Stoffen mit schneller Eliminationskonstante bei mehrfacher Verabreichung zu erheblichen Schwankungen der Plasmaspiegel und kann ein eventuelles temporäres Nachlassen der Wirkung und bei erforderlicher höherer Dosierung auch das eventuelle Auftreten von Nebenwirkungen verursachen.

Eine Verabreichungsform mit verzögerter Freisetzung kann diese Spitzenwerte der Plasmakonzentration vermeiden und eine gleichmäßige Konzentration des Wirkstoffs im Blut gewährleisten. Dies ermöglicht die Verringerung von unerwünschten Effekten, wie z.B. hydroelektronische und stoffwechselartige Störungen, die durch Änderungen des Plasmaspiegels des Wirkstoffs hervorgerufen werden können. Retardierte Darreichungsformen haben den Vorteil, dass sie subtherapeutische und toxische Plasma- bzw. Gewebskonzentrationen vermeiden und stattdessen verbesserte Plasma- bzw. Gewebskonzentrationen über einen längeren Zeitraum aufrecht erhalten. Auf diese Weise kann die Gesamtdosis bei Erzielung einer vergleichbaren Wirkung reduziert werden. Ferner ist meist die Patienten-Compliance verbessert und die Einnahmehäufigkeit kann verringert werden.

Die verzögerte Freisetzung von Indapamid ermöglicht somit einen verbesserten therapeutischen Index. Hierzu ist es erforderlich, eine über die Zeit verzögerte Freisetzung des Wirkstoffs in gesteuerter Form sicherzustellen. Die Freisetzungsrate des Wirkstoffs aus der pharmazeutischen Darreichungsform sollte dabei reproduzierbar sein und mit der Plasmakonzentration korrelieren. Besonders bevorzugt ist eine Freisetzung nullter Ordnung, d.h. eine möglichst linearer Verlauf des Freisetzungsprofils (gesamte Freisetzung gegen Zeit).

Eine Möglichkeit, die gesteuerte, zeitlich verzögerte Wirkstofffreisetzung zu erzielen, beruht auf der Einbettung des Wirkstoffs in einer Matrix, welche aus einem Polymer gebildet wird. Es ist eine Vielzahl von Polymeren beschrieben worden, die zur Bildung einer solchen Matrix eingesetzt werden können, wie z.B. Cellulosederivate, insbesondere Celluloseether wie Hydroxypropylcellulose, Hydroxymethylcellulose, Methylcellulose und Hydroxypropylmethylcellulose.

Tabletten, welche Indapamid zeitlich verzögert freisetzen, sind im Stand der Technik bekannt. Retardtabletten werden beispielsweise in der Bundesrepublik Deutschland unter der Bezeichnung "Natrilix^{®} SR 1,5 mg" vermarktet.

Die Herstellung von Tabletten erfolgt in der Regel entweder durch Verpressen vorbehandelter granulierter Stoffe oder durch Direktverpressung (Direkttablettierung) der Ausgangsstoffe.

Voraussetzung für eine Direkttablettierung, welche ohne vorhergehende Granulation erfolgt, sind eine ausreichende plastische Verformbarkeit der Tablettiermischung, gute Fließeigenschaften und keine Entmischungstendenzen. Diese drei Voraussetzungen zu beherrschen, kann je nach Wirkstoff und Dosis außerordentlich schwierig sein. So können kristalline Substanzen aufgrund ihrer Kristallstruktur einen großen Elastizitätsbereich aufweisen, der erst bei hohen Materialspannungen in eine irreversible Verformung, plastisches Fließen oder Bruch übergeht. Außerdem kann die Anisotropie der Kristalle auf den ganzen Pressling übertragen werden. Bereits geringe Abweichungen bei den Mischungsbestandteilen in Partikelgröße oder Partikelform können zu Tabletten führen, die mechanisch nicht ausreichend fest sind.

Das größte Hindernis für die Direktverpressung stellen damit im allgemeinen die Wirkstoffe dar. Die meisten Arzneistoffe sind nicht direkt tablettierbar. Sie müssen entweder mit direkt komprimierbaren Hilfsstoffen vermischt und auf diese Weise verdünnt werden oder in spezielle, direkt tablettierbare Formen überführt werden. Eine gute Zusammenfassung der Probleme bei der Direkttablettierung kann der Druckschrift Pharma International, 1982, 151-153 entnommen werden. Die Druckschrift erläutert, dass insbesondere bei der Herstellung von Tabletten mit niedrig dosierten Arzneistoffen (d.h. bei Wirkstoffkonzentrationen von weniger als 5 Gew.-% bezogen auf das Gesamtgewicht der Tabletten) Probleme bei der Direktverpressung auftreten können, wie z.B. die irreversible Adsorption an direkt tablettierbare Trägersubstanzen und als Folge davon eine schlechtere Bioverfügbarkeit und Mehrkosten für eine ggf. erforderliche Mikronisierung der Wirkstoffe. Eine weitere Problematik besteht insbesondere darin, eine Wirkstoffgleichverteilung (Content Uniformity) der niedrig dosierten Wirkstoffe zu erreichen. In diesem Fall treten nämlich häufig Entmischungserscheinungen nachteilig in Erscheinung. Diese führen u.a. dazu, dass der Wirkstoff innerhalb der Tablette nicht mehr gleichmäßig verteilt ist, was sich bei Matrixsystemen hinsichtlich der zeitlich verzögerten Freisetzung des Wirkstoffs besonders nachteilig auswirkt. Ferner können die Entmischungserscheinungen dazu führen, dass die einzelnen Tabletten innerhalb einer Charge variierende Wirkstoffgehalte aufweisen. Bei niedrig dosierten Wirkstoffen wird daher in der Regel keine Direkttablettierung durchgeführt, sondern auf Granulierungsverfahren zurückgegriffen, bei denen auch niedrig dosierte Wirkstoffe gleichmäßig verteilt werden.

Entsprechend offenbart EP-A 519 820 ein Granulierungsverfahren zur Herstellung von Tabletten, welche etwa 1 % Indapamid enthalten. Der Wirkstoff wird aus den Tabletten verzögert freigesetzt. Das beschriebene Herstellungsverfahren beruht auf einer Feuchtgranulierung. Zunächst wird eine Mischung aus Indapamid, Polyvidon und Laktose hergestellt, mit einer wässrig-alkoholischen Lösung benetzt und die so erhaltene feuchte Masse anschließend granuliert. Nach der Trocknung und Kalibrierung erfolgt Vermischung des Granulats mit Methylhydroxypropylcellulose, Gleitmittelbehandlung mit kolloidalem Siliziumdioxid und Magnesiumstearat und Verpressung der Mischung auf einer Rotationspressvorrichtung. EP-A 519 820 offenbart, dass bei diesem Verfahren gleichzeitig eine Technik der Feuchtgranulierung und der Direktverpressung angewendet werde, jedoch entspricht die Bedeutung des Begriffs "Direktverpressung" in diesem Sinne nicht der allgemein anerkannten Bedeutung dieses Fachbegriffs. Da nämlich gemäß EP-A 519 820 die Tablette nicht in einem einzigen Kompressionsschritt direkt aus einer pulverförmigen (freifließenden) Mischung aller Ausgangsstoffe hergestellt wird, sondern zunächst die Zwischenstufe der Granulierung durchlaufen wird, handelt es sich bei dem Verfahren um keine Direktverpressung. Bei dem Tablettierverfahren der EP-A 519 820 wird das Indapamid nicht in frei fließender Form sondern als Granulat eingesetzt.

Der mehrstufige Prozess der Feuchtgranulierung, Trocknung und anschließenden Verpressung zu Tabletten, von dem auch EP-A 519 820 Gebrauch macht, ist im Regelfall hinsichtlich seiner Durchführbarkeit relativ unproblematisch. So lassen sich im allgemeinen Feuchtgranulate am sichersten und am komplikationslosesten tablettieren, insbesondere auch bei niedrigdosierten Darreichungsformen. Deshalb ist die Feuchtgranulierung normalerweise das Mittel der Wahl, und Feuchtgranulate sind das übliche Zwischenprodukt bei der Herstellung von Tabletten mit niedrigem Wirkstoffgehalt von weniger als 5%.

Auch G. Damien et al. (Clin. Pharmacokinet. 1999, Vol. 37, Suppl. 1, S. 13-19) offenbart Retard-Präparate mit Indapamid. Im Einklang mit dem allgemeinen Fachwissen offenbart diese Druckschrift, dass es bei der Direktverpressung schwierig ist, eine gute Homogenität der pharmazeutischen Zusammensetzung zu erhalten, wenn ein geringer Wirkstoffgehalt, wie bei Indapamid, verwendet werden soll. Daher wurden die Indapamid-Retardformulierungen, die in dieser Druckschrift beschrieben sind, nicht durch Direktverpressung sondern durch ein übliches Granulationsverfahren hergestellt, und Formulierungen, die zur Direktverpressung geeignet sind, sind dieser Druckschrift nicht zu entnehmen.

GB-A 2 173 399 offenbart Arzneimittel mit einer Kombination von zwei Wirkstoffen mit unterschiedlichen zeitlichen Wirkungsprofilen. Durch eine geeignete Wahl des Wirkstoffverhältnisses kann eine pharmazeutische Zusammensetzung erzeugt werden, die eine gewünschte Plasmakonzentration über einen langen Zeitraum ermöglicht. Bei den Formulierungen der GB-A 2 173 399 handelt es sich ausdrücklich nicht um Retardformulierungen. Die verlängerte Wirkdauer wird nicht durch eine verzögerte Wirkstofffreisetzung verursacht, sondern durch die Kombination zweier Wirkstoffe mit unterschiedlichen zeitlichen Wirkungsprofilen in einer schnell freisetzenden Tablette. Arzneimittelformulierungen zur Direktverpressung werden in der GB-A 2 173 399 nicht offenbart.

Nachteilig bei der Feuchtgranulierung ist, dass besondere Maschinen zur Granulierung eingesetzt werden müssen, Lösungsmittel zur Herstellung der feuchten Masse erforderlich sind und der sich an die Granulierung anschließende Trocknungsschritt zusätzliche Energie erfordert, wodurch auch der Wirkstoff thermischen Einflüssen ausgesetzt wird. Insgesamt ist das Verfahren der Feuchtgranulierung vergleichsweise aufwendig und kostenintensiv. Es besteht auch die Gefahr der Verunreinigung der Tablette durch Lösemittel.

Es besteht daher ein Bedarf an pharmazeutischen Darreichungsformen, welche den Wirkstoff Indapamid verzögert freisetzen und Vorteile gegenüber den Darreichungsformen des Standes der Technik aufweisen. Die pharmazeutischen Darreichungsformen sollten insbesondere einfach und ökonomisch herstellbar sein und dennoch gute Eigenschaften bei der Verabreichung aufweisen. Dazu sollte der Wirkstoff möglichst gleichmäßig innerhalb der pharmazeutischen Darreichungsform verteilt sein, obwohl der Wirkstoffgehalt vergleichsweise gering ist, z.B. 5,0 Gew.-% oder weniger bezogen auf das Gesamtgewicht der Darreichungsform beträgt. Vor allem soll der Wirkstoff auch bei größeren Chargen in den Tabletten gleich verteilt sein, d.h. der Wirkstoffgehalt soll in allen Tabletten möglichst gleich sein, wobei geringe Schwankungen zwischen einzelnen Tabletten nicht vermieden werden können. Diese Schwankungen sollen aber so gering wie möglich sein und auf jeden Fall innerhalb der durch die Arzneibücher (insbesondere die europäische und die US-Pharmakopoe) vorgegebenen entsprechenden Grenzwerte für den Wirkstoff liegen, bevorzugt nicht mehr als ± 10% betragen, insbesondere im Bereich von 95% bis 108%, stärker bevorzugt im Bereich von 96% bis 106% liegen.

Diese Aufgabe wird durch den Gegenstand der Patentansprüche gelöst. Die Erfindung betrifft damit eine pharmazeutische Zusammensetzung, die zur Direktverpressung von Tabletten geeignet ist, umfassend den Wirkstoff Indapamid oder eines seiner pharmazeutisch verträglichen Solvate oder ein Salz des Wirkstoffs oder ein Solvat eines Salzes in freifließender Form, wobei der Gehalt des Wirkstoffs 5,0 Gew.-% oder weniger bezogen auf das Gesamtgewicht der Zusammensetzung beträgt, und einen oder mehrere Matrixbildner umfassend Hydroxypropylmethylcellulose und ein Füllmittel in einer Menge von 45 bis 75 Gew.-%, wobei das Füllmittel agglomerierte Laktose ist.

Die Erfindung betrifft ebenfalls die aus diesen pharmazeutischen Zusammensetzungen durch Direktverpressung erhältlichen Tabletten und das Verfahren, mit dem die Zusammensetzungen zu Tabletten verpresst werden.

Es wurde gefunden, dass eine pharmazeutische Zusammensetzung, welche den freifließenden Wirkstoff Indapamid in einer Menge von 5,0 Gew.-% oder weniger und einen Matrixbildner enthält, direkt zu Tabletten verpresst werden kann, ohne dass zuvor eine Feucht- oder Trockengranulierung erforderlich ist. Überraschenderweise kann der Wirkstoff Indapamid auch in den geforderten sehr niedrigen Konzentrationen von 5% oder weniger in frei fließender Form durch Direktverpressung zu Tabletten verarbeitet werden, die eine ausgezeichnete Freisetzungscharakteristik aufweisen. Die im Stand der Technik beschriebenen Probleme der unzureichenden Wirkstoffgleichverteilung und schlechteren Bioverfügbarkeit treten bei der Direktverpressung von Indapamid überraschenderweise nicht auf. Auf das aufwendige Verfahren der Feuchtgranulierung, wie es beispielsweise aus EP-A 519 820 bekannt ist, kann somit verzichtet werden, ohne dabei Nachteile, insbesondere hinsichtlich der Wirkstofffreisetzung und -verteilung, in Kauf nehmen zu müssen.

Unter Direktverpressung im Sinne dieser Beschreibung ist die Komprimierung einer Mischung zu verstehen, die neben dem freifließenden Wirkstoff noch weitere Hilfsstoffe, wie z.B. Füll-, Binde-, Schmier- sowie ggf. Sprengmittel enthält. Falls erforderlich, können auch Fließregulierungsmittel, Aromastoffe, Süßstoffe, Matrixbildner, etc. hinzukommen. Ein Verfahren, bei dem der freifließende Wirkstoff zunächst granuliert und anschließend verpresst wird, ist keine Direktverpressung im Sinne dieser Beschreibung, eine Definition, die auch der Verwendung des Begriffes Direktverpressung oder Direkttablettierung in der einschlägigen Fachliteratur entspricht.

Die Erfindung betrifft eine pharmazeutische Zusammensetzung, die zur Direktverpressung von Tabletten geeignet ist. Die Zusammensetzung umfasst den Wirkstoff Indapamid oder eines seiner pharmazeutisch verträglichen Solvate oder Salze oder das Solvat eines Salzes in freifließender Form und einen oder mehrere Matrixbildner umfassend Hydroxypropylmethylcellulose. Der Gehalt des freifließenden Wirkstoffs in der pharmazeutischen Zusammensetzung beträgt 5,0 Gew.-% oder weniger, bevorzugter 4,0 Gew.-% oder weniger und insbesondere 3,0 Gew.-% oder weniger, bezogen auf das Gesamtgewicht der Zusammensetzung. Liegt der Wirkstoff nicht in freier Form, sondern als Solvat oder Salz oder Solvat eines Salzes vor, so bezieht sich die Prozentangabe auf das Gewicht der Wirksubstanz als solche, also nicht auf das Gewicht des Solvates oder Salzes oder des Solvats des Salzes. Der Wirkstoffgehalt beträgt bevorzugt 0,001 bis 5,0 Gew.-%, bevorzugter 0,01 bis 2,5 Gew.-%, noch bevorzugter 0,1 bis 1,5 Gew.-%, am bevorzugtesten 0,5 bis 1,0 Gew.-% und insbesondere 0,65 bis 0,85 Gew.-% bezogen auf das Gesamtgewicht der pharmazeutischen Zusammensetzung.

Erfindungsgemäß ist die pharmazeutische Zusammensetzung dazu geeignet, direkt ausgehend von der Pulverform des Wirkstoffs, d.h. nicht über den Umweg der Granulierung, zu Tabletten verpresst zu werden.

Der Wirkstoff Indapamid oder eines seiner pharmazeutisch verträglichen Solvate oder eines Salzes oder eines Solvats des Salzes liegt erfindungsgemäß in freifließender Form vor. Es versteht sich von selbst, dass erfindungsgemäß Salze des Wirkstoffs pharmazeutisch verträgliche Salze sind.

Als Wirkstoffe sind erfindungsgemäß neben wasserfreiem Indapamid dessen pharmazeutisch verträglichen Solvate, insbesondere Hydrate bevorzugt, wobei Indapamid-Hemihydrat besonders bevorzugt ist.

In einer Ausführungsform enthält das erfindungsgemäße Arzneimittel keinen weiteren Wirkstoff, außer Indapamid, insbesondere kein 3-Aminopropoxyindol.

Die erfindungsgemäßen pharmazeutischen Zusammensetzungen enthalten einen oder mehrere Matrixbildner umfassend Hydroxypropylmethylcellulose. Der Matrixbildner dient dazu, den darin eingebetteten Wirkstoff zeitlich verzögert freizusetzen, so dass Tabletten, welche aus den erfindungsgemäßen pharmazeutischen Zusammensetzungen hergestellt werden, eine zeitlich verzögerte Freisetzung des Wirkstoffs gewährleisten.

Geeignete Matrixbildner sind dem Fachmann bekannt. Der Matrixbildner umfasst Hydroxypropylmethylcellulose. Geeignete Hydroxypropylmethylcellulosen sind beispielsweise unter der Bezeichnung "Methocel" bei der Fa. Colorcon erhältlich, wobei die Produkte Methocel K15M, Methocel K4M und Methocel K100LV bzw. deren Kombinationen besonders vorteilhaft sind. Es wird ferner auf Fiedler- Lexikon der Hilfsstoffe, Editio Cantor Verlag Aulendorf, 5. Auflage 2002 verwiesen. Bevorzugt ist auch eine hochviskose Methylhydroxy-propylcellulose, gegebenenfalls im Gemisch mit einem niederviskosen Cellulosederivat wie vorstehend definiert.

Über Menge und Art des verwendeten Matrixbildners kann die Freisetzungsrate des Wirkstoffs aus einer Tablette, welche aus der pharmazeutischen Zusammensetzung hergestellt wird, gesteuert werden. Eine vorgegebene Freisetzungsrate kann so durch einfache Routineversuche vom Fachmann durch die Wahl eines geeigneten Matrixbildners und Variation der Menge des Matrixbildners eingestellt werden. Beispielsweise können Hydroxypropylmethylcellulosen unterschiedlicher Viskosität gemischt werden, und in einer bevorzugten Ausführungsform weisen die erfindungsgemäßen Arzneimittel zumindest zwei Hydroxypropylmethylcellulosen unterschiedlicher Viskosität auf.

Die erfindungsgemäße pharmazeutische Zusammensetzung enthält einen oder mehrere Matrixbildner umfassend Hydroxypropylmethylcellulose. Der Gehalt an Matrixbilder(n) beträgt bevorzugt 5 bis 95 Gew.-%, bevorzugter 10 bis 80 Gew.-%, noch bevorzugter 20 bis 55 Gew.-%, am bevorzugtesten 30 bis 45 Gew.-% und insbesondere 35 bis 40 Gew.-% bezogen auf das Gesamtgewicht der pharmazeutischen Zusammensetzung.

In einer erfindungsgemäß bevorzugten Ausführungsform enthält die pharmazeutische Zusammensetzung kein Polyvinylpyrrolidon, insbesondere kein quervernetztes Polyvinylpyrrolidon (Polyvidon).

Erfindungsgemäß enthält die pharmazeutische Zusammensetzung ein Füllmittel, wobei das Füllmittel agglomerierter Laktose ist. Agglomerierte Laktose ist unter der Bezeichnung "Tablettose" kommerziell erhältlich. Bei Tablettose handelt es sich zwar um Handelsprodukte, deren chemische Identität ist aber einem Fachmann geläufig, und eine genaue Beschreibung dieser Produkte ist z.B. in Fiedler - Lexikon der Hilfsstoffe, Editio Cantor Verlag Aulendorf, 5. Auflage 2002 offenbart, und auf diese Druckschrift wird insoweit ausdrücklich Bezug genommen.

Der Gehalt an Füllmittel(n) in der erfindungsgemäßen pharmazeutischen Zusammensetzung beträgt 45 bis 75 Gew.-%, bevorzugt 50 bis 70 Gew.-% und insbesondere 55 bis 65 Gew.-% bezogen auf das Gesamtgewicht der pharmazeutischen Zusammensetzung.

In einer bevorzugten Ausführungsform der Erfindung enthält die pharmazeutische Zusammensetzung ein oder mehrere Adsorptionsmittel. Geeignete Adsorptionsmittel sind dem Fachmann bekannt, beispielsweise hochdisperses Siliziumdioxid, Stärke, Laktose, Kaolin, Bolus, Bentonit und kolloidale Kieselsäure. Hochdisperses Siliziumdioxid ist besonders bevorzugt und beispielsweise unter der Bezeichnung Aerosil kommerziell erhältlich. Es wird wiederum auf *Fiedler - Lexikon der Hilfsstoffe,* Editio Cantor Verlag Aulendorf, 5. Auflage 2002 verwiesen.

Der Gehalt an Adsorptionsmittel(n) in der erfindungsgemäßen pharmazeutischen Zusammensetzung beträgt bevorzugt 0,001 bis 10,0 Gew.-%, bevorzugter 0,01 bis 5,0 Gew.-%, noch bevorzugter 0,1 bis 2,5 Gew.-%, am bevorzugtesten 0,25 bis 1,0 Gew.-% und insbesondere 0,4 bis 0,6 Gew.-% bezogen auf das Gesamtgewicht der pharmazeutischen Zusammensetzung.

In einer bevorzugten Ausführungsform der Erfindung enthält die pharmazeutische Zusammensetzung ein oder mehrere Gleitmittel (Fließregulierungsmittel). Geeignete Gleitmittel sind dem Fachmann bekannt, beispielsweise Talkum, Magnesiumstearat, Calciumstearat, feste Polyethylenglykole oder Natriumstearylfumarat.

Der Gehalt an Gleitmittel(n) in der erfindungsgemäßen pharmazeutischen Zusammensetzung beträgt bevorzugt 0,001 bis 10,0 Gew.-%, bevorzugter 0,01 bis 5,0 Gew.-%, noch bevorzugter 0,1 bis 2,5 Gew.-%, am bevorzugtesten 0,25 bis 1,0 Gew.-% und insbesondere 0,4 bis 0,6 Gew.-% bezogen auf das Gesamtgewicht der pharmazeutischen Zusammensetzung.

Die erfindungsgemäße Zusammensetzung kann ferner weitere übliche Hilfsstoffe in üblichen Mengen enthalten, beispielsweise Sprengmittel, Feuchthaltemittel, Gegensprengmittel, Resorptionsbeschleuniger, Hydrophilisierungsmittel, Farbstoffe, Süßungsmittel, Aromastoffe, etc. Geeignete Hilfsstoffe und deren geeigneten Gehalte sind dem Fachmann bekannt. Diesbezüglich kann beispielsweise auf Fiedler - Lexikon der Hilfsstoffe, Editio Cantor Verlag Aulendorf, 5. Auflage 2002 verwiesen werden.

Die erfindungsgemäße pharmazeutische Zusammensetzung kann weitere Wirkstoffe enthalten. In einer bevorzugten Ausführungsform enthält die erfindungsgemäße Zusammensetzung jedoch als Wirkstoff ausschließlich Indapamid bzw. eines seiner pharmazeutisch verträglichen Solvate oder Salze oder ein Solvat eines Salzes.

Die pharmazeutische Zusammensetzung enthält Indapamid und/oder ein Indapamid-Solvat, insbesondere Indapamid-Hemihydrat als Wirkstoff, Hydroxypropylmethylcellulose als Matrixbildner und ein Füllmittel, wobei das Füllmittel agglomerierte Laktose (insbesondere Tablettose) ist. Am bevorzugtesten enthält die pharmazeutische Zusammensetzung zusätzlich ein Adsorptionsmittel und/oder ein Gleitmittel, wobei das Adsorptionsmittel bevorzugt hochdisperses Siliziumdioxid und das Gleitmittel bevorzugt Magnesiumstearat ist.

In einer bevorzugteren Ausführungsform enthält die pharmazeutische Zusammensetzung Indapamid oder ein Indapamid-Solvat, insbesondere Indapamid-Hemihydrat, eine oder mehrere verschiedene Hydroxypropylmethylcellulosen (HPMC), ein Füllmittel, das agglomerierter Laktose ist, und optional hochdisperses Siliziumdioxid und/oder Magnesiumstearat, wobei die bevorzugten relativen Mengenverhältnisse in folgender Tabelle zusammengestellt sind:

| | [Gew.-%] |
|---|---|
| Inhaltsstoff | bevorzugt |
| Indapamid oder Indapamid-Solvat, insbesondere Indapamid-Hemihydrat | 0,5 bis 1,0 |
| HPMC | 30,0 bis 50,0 |
| Füllmittel (Laktose wie vorstehend definiert) | 50,0 bis 70,0 |
| hochdisperses Siliziumdioxid | 0,01 bis 1,0 |
| Magnesiumstearat | 0,01 bis 1,0 |

Die Erfindung betrifft auch eine pharmazeutische Darreichungsform, welche durch Direktverpressung der oben beschriebenen pharmazeutischen Zusammensetzung erhältlich sind. Die pharmazeutische Darreichungsform ist bevorzugt eine Tablette, welche eine verzögerte Freisetzung des darin enthaltenen Wirkstoffs Indapamid (bzw. eines seiner pharmazeutisch verträglichen Solvate oder Salze oder ein Solvat eines derartigen Salzes) gewährleistet.

Die Erfindung betrifft auch ein Verfahren zur Herstellung einer Tablette mit verzögerter Wirkstofffreisetzung, wobei das Verfahren den Schritt der Direktverpressung der oben beschriebenen pharmazeutischen Zusammensetzung umfasst.

Direktverpresste Tabletten können z.B. hergestellt werden, indem geeignete Mengen der verschiedenen Bestandteile gesiebt, anschließend in einem üblichen Mischer zu einem homogenen frei fließenden Pulver gemischt werden und dann mittels einer üblichen Tablettiermaschine verpresst werden.

Vorrichtungen zur Direktverpressung von pharmazeutischen Zusammensetzungen sind im Handel erhältlich. Die erfindungsgemäßen pharmazeutischen Darreichungsformen in Form von Tabletten lassen sich beispielsweise durch Direktverpressung der erfindungsgemäßen Zusammensetzung auf einer Fette 1200 Rundläuferpresse herstellen.

Die erfindungsgemäßen Tabletten können mit einem Überzug beschichtet sein. Geeignete Filmbildner sind dem Fachmann bekannt. Es wird ferner auf Fiedler - Lexikon der Hilfsstoffe, Editio Cantor Verlag Aulendorf, 5. Auflage 2002 verwiesen. In einer bevorzugten Ausführungsform sind die Tabletten auf übliche Art und Weise beschichtet, z. B. mit einem Film überzogen, der sie vor Feuchtigkeit schützen und/oder die Einnahme erleichtern soll.

Bevorzugt beträgt das Gesamtgewicht der erfindungsgemäßen Tabletten 25 bis 500 mg, bevorzugter 50 bis 400 mg, noch bevorzugter 100 bis 300 mg, am bevorzugtesten 125 bis 275 mg und insbesondere 150 bis 250 mg. Bevorzugt sind die Tabletten zur einmaligen täglichen Verabreichung bestimmt.

Bevorzugt enthalten die erfindungsgemäßen Tabletten Indapamid bzw. eines seiner pharmazeutisch verträglichen Solvate oder Salze oder ein Solvat eines Salzes in einer Gesamtmenge von 0,1 bis 3,0 mg, bevorzugter 0,5 bis 2,5 mg, am bevorzugtesten 1,0 bis 2,0 mg und insbesondere 1,4 bis 1,6 mg.

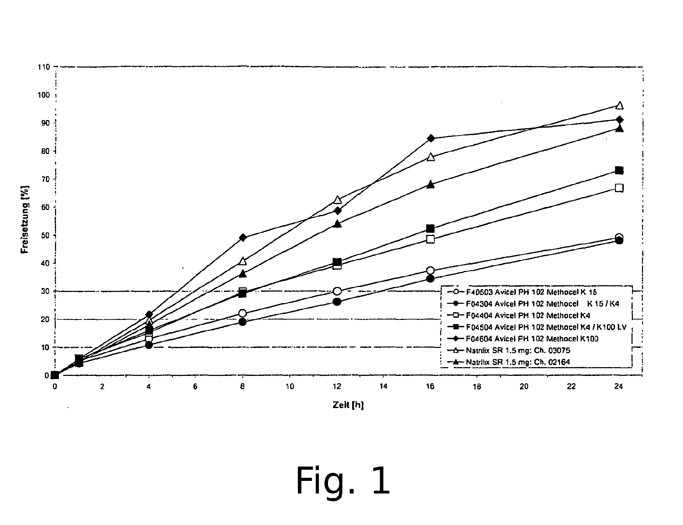

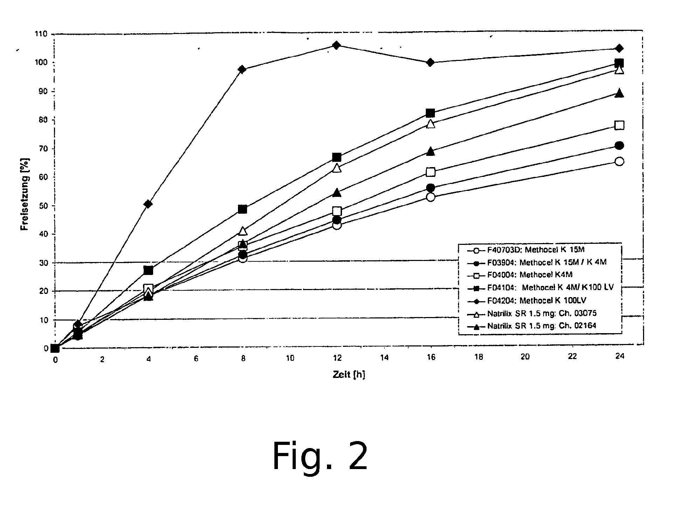

Erfindungsgemäß bevorzugt ist die Freisetzung in etwa nullter Ordnung, d.h. es gibt bis zur vollständigen Freisetzung einen in etwa linearen Zusammenhang zwischen der prozentualen Freisetzung und der Zeit, wie in den Figuren 1 und 2 gezeigt wird. Bevorzugt weisen die erfindungsgemäßen Tabletten eine Freisetzungscharakteristik auf, bei der die Freisetzung während mehr als 8 Stunden linear verläuft. Bevorzugt werden etwa 50% der Gesamtmenge des Indapamids zwischen 5 und 24 Stunden, stärker bevorzugt zwischen 5 und 18 Stunden, freigesetzt. Bevorzugt erfolgt die Freisetzung ferner so, dass sie beim Menschen zu Blutspiegeln zwischen 20 und 80 ng/ml höchstens 24 Stunden nach der Verabreichung der Tablette führt. Die Einheitsdosierung kann dabei in Abhängigkeit von dem Alter und dem Gewicht des Patienten und der Art und der Schwere der Erkrankung variieren. Bevorzugt erstreckt sie sich zwischen 1 und 2,5 mg täglich. Die Bestimmung der Freisetzung erfolgt, wie auch in der EP-A 519 820, auf übliche Art und Weise. Beispielhaft ist die Bestimmung der Freisetzung des Wirkstoffs auch in den nachstehenden Beispielen näher beschrieben.

Die erfindungsgemäßen Tabletten weisen bevorzugt eine durch diametrale Zerstörung gemessene Härte von 40 bis 180 N, bevorzugter 100 bis 160 N und insbesondere von 120 bis 150 N auf. Die Härte der erfindungsgemäßen Tabletten kann mit herkömmlichen Methoden gemessen werden. Diesbezüglich kann beispielsweise auf das Europäische Arzneibuch, 4. Ausgabe, Grundwerk 2002, Abschnitt 2.9.8 "Bruchfestigkeit von Tabletten" verwiesen werden. Die Bruchfestigkeit wurde im Rahmen dieser Anmeldung mit einem ERWEKA TBH20 bestimmt, sofern nichts anderes angegeben oder offensichtlich ist.

Sofern im Rahmen dieser Beschreibung nichts anderes angegeben oder offensichtlich ist, beziehen sich Prozentangaben immer auf Gewichtsprozent auf Grundlage des Gesamtgewichts der angesprochenen Darreichungsform.
Figur 1 zeigt die in vitro Freisetzung verschiedener pharmazeutischer Zusammensetzungen, welche als Füllmittel Avicel PH102 enthalten. Zum Vergleich sind die entsprechenden Daten für kommerziell erhältliche Natrilix Präparate dargestellt.
Figur 2 zeigt die in vitro Freisetzung verschiedener erfindungsgemäßer pharmazeutischer Zusammensetzungen, welche als Füllmittel Tablettose enthalten. Zum Vergleich sind die entsprechenden Daten für kommerziell erhältliche Natrilix Präparate dargestellt.
Figur 3 zeigt die Gleichförmigkeit des Gehalts (Content Uniformity, CUT) erfindungsgemäßer Chargen, welche als Scale-up-Chargen untersucht wurden.

Die nachfolgenden Beispiele dienen zur näheren Erläuterung der Erfindung.

Alle nachfolgenden Rezepturen enthalten HPMC der Fa. Colorcon (Methocel). Hierbei wurden verschiedene Qualitäten mit verschiedenen Viskositäten kombiniert, um die Freisetzung zu beeinflussen. Als Füllmittel wurden Avicel PH102 (mikrokristalline Cellulose) oder Tablettose (agglomerierte Laktose) verwendet. Alle nachfolgend aufgeführten Chargen wurden durch Direkttablettierung hergestellt:

### Beispiel 1 (nicht erfindungsgemäß):

**Rezepturen mit Avicel als Füllmittel:**

| Substanz | F40503 | F04304 | F04404 | F04504 | F04604 |
|---|---|---|---|---|---|
| Indapamid-Hemihydrat | 1,50 | 1,50 | 1,50 | 1,50 | 1,50 |
| Methocel K15M | 77,36 | 38,68 | - | - | - |
| Methocel K4M | - | 38,68 | 77,36 | 38,68 | - |
| Methocel K100LV | - | - | - | 38,68 | 77,36 |
| Avicel PH102 | 119,14 | 119,14 | 119,14 | 119,14 | 119,14 |
| Aerosil | 1,00 | 1,00 | 1,00 | 1,00 | 1,00 |
| Magnesiumstearat | 1,00 | 1,00 | 1,00 | 1,00 | 1,00 |
| Masse Tablette [mg] | 200,00 | 200,00 | 200,00 | 200,00 | 200,00 |

### Beispiel 2:

Rezepturen mit Tablettose 80 (α-Laktose-Monohydrat, agglomeriert, zur Direkttablettierung, Firma Meggle, Wasserburg, Deutschland) als Füllmittel:

| Substanz | F40703 F16904 | F03904 | F04004 | F04104 F08204 | F04204 F17004 |
|---|---|---|---|---|---|
| Indapamid-Hemihydrat | 1,50 | 1,50 | 1,50 | 1,50 | 1,50 |
| Methocel K15M | 77,36 | 38,68 | - | - | - |
| Methocel K4M | - | 38,68 | 77,36 | 38,68 | - |
| Methocel K100LV | - | - | - | 38,68 | 77,36 |
| Tablettose | 119,14 | 119,14 | 119,14 | 119,14 | 119,14 |
| Aerosil | 1,00 | 1,00 | 1,00 | 1,00 | 1,00 |
| Magnesiumstearat | 1,00 | 1,00 | 1,00 | 1,00 | 1,00 |
| Masse Tablette [mg] | 200,00 | 200,00 | 200,00 | 200,00 | 200,00 |

Die in Beispielen 1 und 2 angegebenen Formulierungen wurden nach folgendem Verfahren hergestellt:
Die Gesamtmenge des Indapamid-Hemihydrats wird zunächst mit ca. 4% des Füllmittels (Tablettose oder Avicel) im Beutel oder per Freifall gemischt. Anschließend werden nochmals ca. 20% des Füllmittels (Tablettose oder Avicel) durch 10-minütige Freifallmischung untergemischt. Über ein 1,1 mm Lochsieb werden separat dieses Gemisch, weitere ca. 25% des Füllmittels zusammen mit dem Methocel und der restliche Anteil an Füllmittel gesiebt. Die gesiebten Bestandteile werden 10 Minuten bei 80 U/min in einem Zwangsmischer (bzw. einem Schnell- oder Intensivmischer) gemischt. Anschließend wird gesiebtes Aerosil zugesetzt und gemischt (Freifall, 10 Minuten) und gesiebtes Magnesiumstearat zugesetzt und gemischt (Freifall, 2 Minuten). Es wird auf einer Fette 1200 Rundläufertablettenpresse verpresst.

### Beispiel 3:

In vitro Freisetzung der Füllmittel-Varianten:

Die Freisetzung des Wirkstoffs wurde in einer USP 23 Paddle Apparatur bei einer Temperatur von 37°C ± 0.5°C und einer Rührgeschwindigkeit von 75 U/min bestimmt. Als Prüfmedium wurde ein Phosphatpuffer pH 6,8 nach USP verwendet, der durch Auflösen von 6,8 g Kaliumdihydrogenphosphat und 0,89 g Natriumhydroxid in 1 I demineralisiertem Wasser hergestellt wurde. Der pH-Wert wurde, falls erforderlich, mit 27%-iger Natronlauge auf 6,8 eingestellt. Zur Überprüfung der Freisetzung wurde eine Tablette in 500 ml Prüfmedium vermessen, und es wurden Proben nach 1, 4, 8, 12, 16 und 24 Stunden gezogen. Die Auswertung erfolgte über HPLC unter Verwendung einer Referenzstammlösung aus Indapamid-Hemihydrat in Methanol. Alle verwendeten Reagenzien waren von der Qualität PA oder besser. Die Ergebnisse der Freisetzungsmessungen sind in Figur 1 und Figur 2 gezeigt.

### Beispiel 4:

Gleichförmigkeit der Wirkstoffverteilung:
Die Chargen F08204, F16904 und F17004 stellen Scale-up-Chargen dar. Diese Chargen umfassten jeweils 75.000 Tabletten und wurden auf einer Fette 1200 Rundläuferpresse hergestellt. Von den Endmischungen sowie während der Tablettierung wurden Proben genommen und unter dem Aspekt CUT (Content Uniformity) untersucht.

Der Gehalt an Indapamid wurde über HPLC bestimmt. Hierzu wurde ein ganze Tablette genau gewogen und in einem 50 ml Braunglasmesskolben mit ca. 40 ml Acetonitril/demin. Wasser 8/2 (V/V) versetzt und 10 Minuten im Ultraschallbad behandelt. Nach dem Temperieren auf 20°C wird mit dem Lösemittel bis zur 50 ml Marke aufgefüllt. Nach dem Durchmischen wird die Suspension zentrifugiert. Das klare Zentrifugat wird als Probelösung verwendet. Es wird gegen eine Referenzlösung von Indapamid-Hemihydrat gemessen.

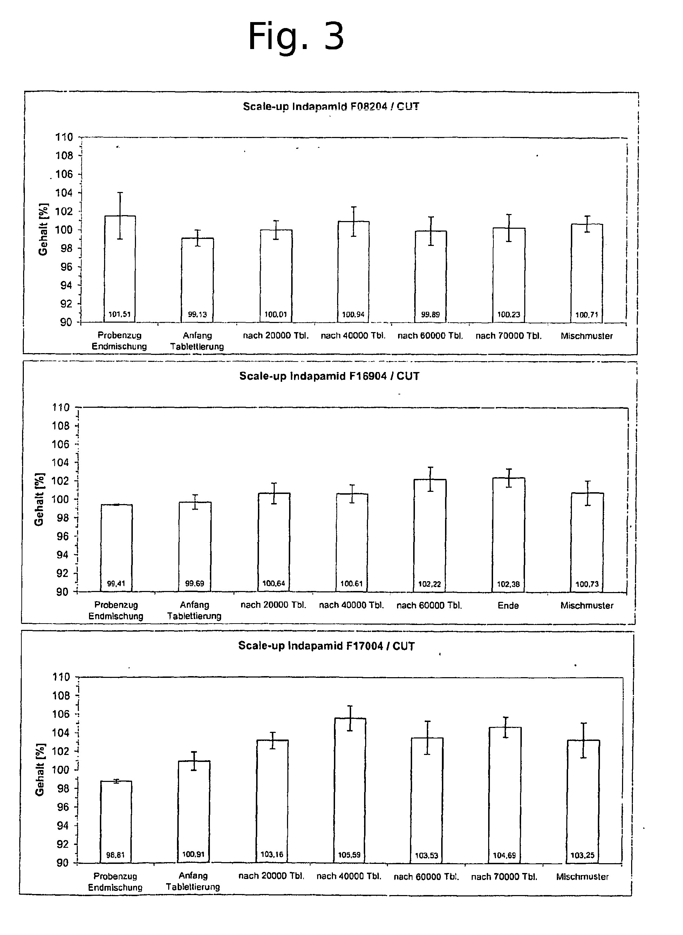

Die Ergebnisse sind in Figur 3 zusammengefasst.

## Patentansprüche

1. Pharmazeutische Zusammensetzung, die zur Direktverpressung von Tabletten mit verzögerter Wirkstofffreisetzung geeignet ist, umfassend den Wirkstoff Indapamid oder eines seiner pharmazeutisch verträglichen Solvate oder Salze oder ein Solvat eines Salzes in freifließender Form, wobei der Gehalt des Wirkstoffs 5,0 Gew.-% oder weniger bezogen auf das Gesamtgewicht der Zusammensetzung beträgt, einen oder mehrere Matrixbildner, umfassend Hydroxypropylmethylcellulose und ein Füllmittel in einer Menge von 45 bis 75 Gew.-%, wobei das Füllmittel agglomerierte Laktose ist.

2. Pharmazeutische Zusammensetzung nach Anspruch 1, wobei die pharmazeutische Zusammensetzung als Matrixbildner zwei oder mehrere Hydroxypropylmethylcellulosen unterschiedlicher Viskosität enthält.

3. Pharmazeutische Zusammensetzung nach einem der vorstehenden Ansprüche, **dadurch gekennzeichnet, dass** der Gehalt des Wirkstoffs 0,1 bis 1,5 Gew.-% bezogen auf das Gesamtgewicht der Zusammensetzung beträgt.

4. Pharmazeutische Zusammensetzung nach einem der vorstehenden Ansprüche, **dadurch gekennzeichnet, dass** sie hochdisperses Siliziumdioxid enthält.

5. Pharmazeutische Zusammensetzung nach einem der vorstehenden Ansprüche, **dadurch gekennzeichnet, dass** sie Magnesiumstearat enthält.

6. Verfahren zur Herstellung einer Tablette mit verzögerter Wirkstofffreisetzung umfassend die Direktverpressung einer pharmazeutischen Zusammensetzung nach einem der Ansprüche 1 bis 5.

7. Tablette erhältlich nach dem Verfahren nach Anspruch 6.

8. Tablette nach Anspruch 7, **dadurch gekennzeichnet, dass** die Freisetzung des Wirkstoffs nach 12 h weniger als 100 % beträgt.

## Claims

1. A pharmaceutical composition suitable for direct compression of tablets with delayed release of the active ingredient comprising the active ingredient indapamide or one of its pharmaceutically acceptable solvates or salts or a solvate of a salt in free-flowing form, wherein the content of the active ingredient is 5.0% by weight or less based on the total weight of the composition, one or more matrix forming agents comprising hydroxypropyl methyl cellulose, and a filler in an amount of from 45 to 75% by weight, wherein the filler is agglomerated lactose.

2. The pharmaceutical composition according to claim 1, wherein the pharmaceutical composition contains as a matrix forming agent two or more hydroxypropyl methyl celluloses of different viscosity.

3. The pharmaceutical composition according to any one of the preceding claims, **characterized in that** the content of active ingredient is 0.1 to 1.5% by weight based on the total weight of the composition.

4. The pharmaceutical composition according to any one of the preceding claims, **characterized in that** it contains highly disperse silica.

5. The pharmaceutical composition according to any one of the preceding claims, **characterized in that** it contains magnesium stearate.

6. A method for preparation of a tablet with delayed release of the active ingredient comprising the direct compression of a pharmaceutical composition according to any one of claims 1 to 5.

7. A tablet obtainable according to the method according to claim 6.

8. The tablet according to claim 7, **characterized in that** the release of the active ingredient is less than 100% after 12h.

## Revendications

1. Composition pharmaceutique qui est appropriée pour la compression directe de comprimés à libération de principe actif différée, comprenant le principe actif indapamide ou l'un de ses solvates ou sels pharmaceutiquement acceptables ou un solvate d'un sel sous forme libre, la teneur en principe actif étant de 5,0 % en poids ou moins par rapport au poids total de la composition, un ou plusieurs formateurs de matrice, comprenant de l'hydroxypropylméthylcellulose et un agent de charge en une quantité de 45 à 75 % en poids, la substance de charge étant du lactose aggloméré.

2. Composition pharmaceutique selon la revendication 1, la composition pharmaceutique contenant comme formateur de matrice, deux hydroxypropylméthylcelluloses ou plus, présentant des viscosités différentes.

3. Composition pharmaceutique selon l'une des revendications précédentes, **caractérisée en ce que** la teneur en substance active est de 0,1 à 1,5 % en poids par rapport au poids total de la composition.

4. Composition pharmaceutique selon l'une des revendications précédentes, **caractérisée en ce qu'**elle contient du dioxyde de silicium hautement dispersé.

5. Composition pharmaceutique selon l'une des revendications précédentes, **caractérisée en ce qu'**elle contient du stéarate de magnésium.

6. Procédé de production d'un comprimé à libération différée de principe actif, comprenant la compression directe d'une composition pharmaceutique selon l'une des revendications 1 à 5.

7. Comprimé pouvant être obtenu selon le procédé selon la revendication 6.

8. Comprimé selon la revendication 7, **caractérisé en ce que** la libération du principe actif est inférieure à 100 % après 12 heures.
